Europäisches Patentamt

European Patent Office

Office européen des brevets

(11) Publication number: 0 366 612
A2

# EUROPEAN PATENT APPLICATION

(21) Application number: 89810798.2

(22) Date of filing: 23.10.89

(51) Int. Cl.5: **C12N 5/00 , C12P 21/08 , B01D 15/08 , C12Q 1/68 , C12N 15/29 , C07K 3/20**

The applicant has filed a statement in accordance with Rule 28 (4) EPC (issue of a sample only to an expert). Accession number(s) of the deposit(s): ATCC HB-9885, HB-9886 and HB-9887.

The microorganism(s) has (have) been deposited with American Type Culture Collection under number(s) ATCC HB-9885, HB-9886 and HB-9887.

(30) Priority: 27.10.88 US 263618

(43) Date of publication of application:
**02.05.90 Bulletin 90/18**

(84) Designated Contracting States:
**AT BE CH DE ES FR GB GR IT LI LU NL SE**

(71) Applicant: **CIBA-GEIGY AG**
**Klybeckstrasse 141**
**CH-4002 Basel(CH)**

Applicant: **UNIVERSITY OF GEORGIA RESEARCH FOUNDATION, INC.**
**616 GSRC**
**Athens, GA 30602(US)**

(72) Inventor: **Cordonnier, Marie-Michele**
**2731 Old Sugar Road**
**Durham, NC 27707(US)**
Inventor: **Pratt, Lee H.**
**390 Snapfinger Drive**
**Athens, GA 30605(US)**
Inventor: **Stewart, Sandy**
**1112 North Gregson Street**
**Durham, NC 27701(US)**
Inventor: **Montoya, Alice**

**deceased(US)**

(74) Representative: **Roth, Bernhard M. et al**
**Patentabteilung der CIBA-Geigy AG**
**Klybeckstrasse 141**
**CH-4002 Basel(CH)**

(54) **Purification of green-plant phytochrome protein and cloning of green-phytochrome genes.**

(57) The present invention provides a novel method to purify green-plant phytochrome; the production of hybridoma cell lines secreting monoclonal antibodies specific for green-plant phytochrome and that do not cross-react significantly with etiolated-plant phytochrome; a partial amino acid sequence for an immunoaffinity-purified green-plant phyto. chrome; oligonucleotide probes for cloning green-phytochrome genes from plant genomic DNA and cDNA libraries; and cloned green-phytochrome genes and cDNA.

# PURIFICATION OF GREEN-PLANT PHYTOCHROME PROTEIN AND CLONING OF GREEN-PHYTOCHROME GENES

The present invention relates to the purification of phytochrome from green (i.e., light-grown) plants, the production of hybridoma cell lines that produce monoclonal antibodies that react selectively with green-plant phytochrome, and the identification of green-phytochrome genes and cDNA.

Phytochrome is a photoreceptor for photomorphogenic responses in plants. That is, phytochrome detects the natural radiation of the plant environment and regulates adaptive growth and developmental responses critical for composition and survival. Phytochrome exists in two photointerconvertible forms: a red-light-absorbing form (Pr) and a far-red-light-absorbing form (Pfr). Thereby, the phytochrome system detects the ratio of red light (R) relative to far-red light (FR) and initiates appropriate morphological adaptation. For example, a low R:FR ratio could indicate the presence of shade from neighboring plants and initiate competitive morphological responses (e.g., increased shoot extension rate).

Historically, most of the understanding of phytochrome derived from the study of this chromoprotein as isolated from etiolated (i.e., dark-grown) plants, rather than from green (i.e., light grown) plants. This is because phytochrome is relatively abundant in etiolated plants, while it is present in green plants in about 50-fold lower quantity. Further, the presence of chlorophyll in green plants makes the spectral assay of phytochrome impractical in green tissues.

Recent studies on phytochrome from green plants have indicated that it is different from phytochrome from etiolated plants. It has been hypothesized that green phyto. chrome and etiolated phytochrome could derive from different genes. Phytochrome of the type that is most abundant in etiolated plants is referred to herein by the term etiolated phytochrome, and, conversely, that which is most abundant in green plants is referred to herein by the term green phytochrome. However, the use of these terms is not meant to imply that there is no green phytochrome in etiolated plants, nor that there is no etiolated phytochrome in green plants. In fact, it has been observed in oat plants that etiolated plants contain a small amount of green phytochrome (Shimazaki Y and Pratt LH, 1985).

Monoclonal antibodies directed to etiolated-oat phytochrome have been previously obtained and it has been demonstrated that most do not bind green-oat phytochrome (Tokuhisa JG, et al, 1985; Shimazaki Y and Pratt LH, 1985 ). One monoclonal antibody directed to etiolated-pea phytochrome (Pea-25) is able to cross-react with at least some of the phytochrome from green oat shoots. The production of monoclonal anti-bodies specific for green phytochrome and that do not cross-react significantly with etiolated phytochrome has proven to be difficult because of the fact that green phytochrome is a very low abundance chromoprotein, estimated to be about 0.002 % of extractable protein. Moreover, it is a very labile protein, and prior purification attempts yielded a relatively poor immunogen. Apparently, immunodominant contaminants were present in the prior method for green phytochrome purification and generated monoclonal antibodies directed primarily to the immunodominant contaminantes.

Two hybridomas (designated GO-1 and GO-2) that were previously reported by the Applicants of the present invention [Pratt LH and Cordonnier M-M (1987)] were prepared by immunizing with partially-purified green-phytochrome immunogen that was purified through hydroxyapatite chromatography as described by Shimazaki and Pratt (1985), and additionally purified by DEAE chromatography prior to electrophoretic purification. However, the GO-1 and GO-2 hybridoma cell lines have proven to be not sufficiently specific for green phytochrome to serve as the needed probes for resolution of phytochrome into individual species and identification of phytochrome proteins encoded by separate phytochrome genes. GO-1 by direct ELISA tested against etiolated phytochrome vs green phytochrome reacted better with etiolated phytochrome than green phytochrome. The ELISA data for GO-2 indicated that its activity was no more specific than a non-immune mouse control.

Resolution of phytochrome into individual species and isolation of the separate genes postulated to encode for the separate species of phytochrome is dependent upon the development of methods for purifying green phytochrome and the production of hybridoma cell lines which produce monoclonal antibodies that are selective for green phytochrome. In order to characterize green phytochrome fully, as well as to do so independently of etiolated phytochrome, it is important to obtain antibodies that are directed to green phytochrome, that do not cross-react significantly with etiolated phytochrome, and that react with green phytochrome sufficiently well to permit their use in sensitive quantitative assays such as ELISA, and for immunopurification.

Accordingly, the present invention addresses a number of objectives:

a) A method for purification of green phytochrome that improves purity and eliminates immunodominant contaminants;

2

b) Production of hybridoma cell lines secreting monoclonal antibodies that are specific for green phytochrome, that do not cross-react significantly with etiolated phytochrome, and that react well with green phytochrome that is undenaturated, as well as denatured.

c) Immunoaffinity purification of green phytochrome and protein sequence analysis of immunoaffinity-purified green phytochrome to demonstrate unequivocally for the first time that green phytochrome is encoded by a separate gene (or genes) than that which encodes for etiolated phytochrome.

d) Design of oligonucleotide probes for green phytochrome genes based on the protein sequence of immunoaffinity-purified green phytochrome.

e) Cloning and purification of green phytochrome promoters and structural genes from genomic libraries for various plants; and cloning green-phytochrome cDNAS from cDNA libraries for plants.

In particular, the present invention relates to a method for purification of green-plant phytochrome that improves purity and eliminates immunodominant contaminants, which method comprises:
- extracting in a suitable extraction buffer plant material from green plants after homogenisation;
- fractionating the resulting crude phytochrome preparation by addition of poly (ethylenimine) and ammonium sulfate;
- further fractionating the resulting phytochrome preparation by hydroxyapatite (HA) chromatography; -
adding one ore more further ammonium sulfate fractionation steps; and
- electrophoresing the resulting phytochrome preparation.

The present invention further relates to hybridoma cell lines that produce monoclonal antibodies that react selectively with a green-plant phytochrome and do not cross-react significantly with etiolated phytochrome from the same plant.

Preferred within the scope of the present invention are hybridoma cell lines that produce monoclonal antibodies that react selectively with a green-plant phytochrome from an oat plant and do not cross-react significantly with etiolated phytochrome from the same plant.

Furhter comprised are hybridoma cell lines that produce monoclonal antibodies that react selectively with a green-plant phytochrome, preferably with a green-plant phytochrome from an oat plant having the partial amino acid sequence:
V-A-E-I-T-G-L-P-T-M-E-A-I-G-M-P-L-V-D.

Especially preferred are the hybridoma cell lines GO-5, GO-7 or GO-8 that produces monoclonal antibodies that react selectively with a green-plant phytochrome.

The present invention further relates to monoclonal antibodies that selectively bind to a green-plant phytochrome, preferably to a green-plant phytochrome from an oat plant and do not cross-react significantly with etiolated phytochrome from the same plant.

Also comprised by the present invention are monoclonal antibodies that selectively bind to a green-plant phytochrome, preferably to a green-plant phytochrome from an oat plant having the partial amino acid sequence:
V-A-E-I-T-G-L-P-T-M-E-A-I-G-M-P-L-V-D.

Especially preferred are monoclonal antibodies produced by a hybridoma cell line identified as GO-5, GO-7 or GO-8.

A further embodiment of the present invention comprises a method for producing hybridoma cell lines that produce mono clonal antibodies that react selectively with a green-plant phytochrome, preferably with a green-plant phytochrome from an oat plant and do not cross-react significantly with etiolated phytochrome from the same plant, wherein as an immunogen a green-plant phytochrome or a fragment therof having the partial amino acid sequence:
V-A-E-I-T-G-L-P-T-M-E-A-I-G-M-P-L-V-D
is used.

Also comprised are hybridoma cell lines that produce monoclonal antibodies that react selectively with a green-plant phytochrome, preferably with a green-plant phytochrome from an oat plant as well as the monoclonal antibodies produced by the said hybridoma cell lines.

Likewise embraced by the present invention is a method for producing monoclonal antibodies that selectively bind to a green-plant phytochrome, preferably to a green-plant phytochrome from an oat plant and most preferably to a green-plant phytochrome from an oat plant having the partial amino acid sequence:
V-A-E-I-T-G-L-P-T-M-E-A-I-G-M-P-L-V-D
and do not cross-react significantly with etiolated phytochrome from the same plant, which method comprises:

(a) *in vitro* or *in vivo* cultivation of a hybridoma cell line, which is capable of producing the above monoclonal antibodies by methods known in the art; and

(b) isolating the monoclonal antibodies produced.

Likewise part of the present invention is a method for purifying a green-plant phytochrome suitable for use as an immunogen to produce hybridomas that react selectively with a green-plant phytochrome comprising purifying a green-plant phytochrome by immunoaffinity chromatography utilizing monoclonal antibodies produced by at least one of the above characteriszed hybridomas.

Using as an immunogen a green-plant phytochrome, preferably a green-plant phytochrome from an oat plant, purified by the above method, a hybridoma cell line that produces monoclonal antibodies that react selectively with a green-plant phytochrome can be readily prepared. This method is also a part of the present invention, as is the hybridoma cell line resulting from this method.

A further embodiment of the present invention comprises an oligonucleotide probe comprising a base sequence selected from the group of base sequences derived from the following base sequence with random base insertions of permissible base substitutions as designated:

```
CCA ACA ATG GAG GCA ATT GGA ATG CC
..T ..T         ..A ..T ..C ..T
..C ..C         ..C ..A ..G
..G ..G         ..G     ..C
```

as well as a mixture of oligonucleotide probes comprising the above designated base sequences.

Preferred within the scope of the present invention is an oligonucleotide probe comprising a base sequence selected from the group of base sequences derived from the following base sequence with random base insertions ofpermissible base substitutions as designated:

```
CCA ACA ATG GAG GCA ATT GGA ATG CC
..T ..T         ..A ..T ..C ..T
      ..C                   ..G
                            ..C
```

as well as a mixture of oligonucleotide probes comprising the above designated base sequences.

The above identified oligonucleotide probes can be used in a method for cloning green-plant phytochrome genomic DNA sequences or green-plant phytochrome genes or fragements thereof that hybridize with the above oligonucleotide probes or for cloning green-plant cDNA that encodes for green-plant phytochrome or a fragment thereof and that hybridize with the above oligonucleotide probes comprising screening genomic DNA or cDNA prepared from a green plant, preferably from an oat plant, with the said oligonucleotide probes.

Methods for cloning green-plant phytochrome genes or fragements thereof or for cloning green-plant cDNA that encodes for green-plant phytochrome or a fragment thereof as well as the genes and cDNAS resulting therefrom are likewise comprised by the present invention.

Preferred are green-plant phytochrome genes or fragments thereof or green-plant phytochrome promoter sequences associated with green-plant phytochrome genes that hybridize to an oligonucleotide probe comprising a base sequence selected from the group of base sequences derived from the following base sequence with random base insertions of permissible base substitutions as designated:

```
CCA ACA ATG GAG GCA ATT GGA ATG CC
..T ..T         ..A ..T ..C ..T
..C ..C         ..C ..A ..G
..G ..G         ..G     ..C
```

as well as to a mixture of oligonucleotide probes comprising the above designated base sequences.

Especially preferred are green-plant phytochrome genes or fragments thereof or green-plant phytochrome promoter genes associated with green-plant phytochrome genes that hybridize to an oligonucleotide probe comprising a base sequence selected from the group of base sequences derived from the following base sequence with random base insertions of permissible base substitutions as designated:

```
CCA ACA ATG GAG GCA ATT GGA ATG CC
..T ..T         ..A ..T ..C ..T
    ..C                     ..G
                            ..C
```

as well as to a mixture of oligonucleotide probes comprising the above designated base sequences.

For purification of green-plant phytochrome plant material from green plants, preferably from oat plants, is extracted in a suitable extraction buffer. The resulting homogenate is further purified by poly (ethylenimine) and ammonium sulfate fractionation precipitating the crude phytochrome preparation with an amount of ammonium sulfat not exceeding approximately 36% by volume of the final concentration of the total preparation. The resulting precipitate is dissolved in a small volume of a resuspension buffer and further fractionated by hydroxyapatite (HA) chromatography, and one or more further ammonium sulfate fractionation steps. Throughout the purification process a protease inhibitor is used. A suitable protease inhibitor, which is preferably used within the inventive process, is iodoacetamide. The HA-purified phytochrome can be further separated by electro-phoresis. Preferred within the scope of the invention is a SDS Polyacrylamidgelelectrophoresis (PAGE). The location of the phytochrom can be identified by its $Zn^{2+}$-induced fluorescence. After staining with a suitable stain, such as, for example, Coomassie blue, phytochrome bands can be excised, stacked in the sample well of a second SDS gel, preferably 7-8 at a time, electrophoresed, transferred to nitrocellulose and visualized first by $Zn^{+2}$-induced fluorescence followed by staining with a suitable stain, preferably with Ponceau S.

For the preparation of an immunisation solution the phytochrome preparation is further purified by excising phytochrome-containing bands from the second SDS gel, which are subsequently washed, excised, washed extensively with water, dried, and dissolved in a suitable solvent, such as, for example, dimethylsulfoxide. The resulting solution is then emulsified by addition of a suitable adjuvant. Preferred within the scope of the present invention is a Freund's adjuvant.

In a preferred embodiment of the present invention, which is described fully in Example I below, green phytochrome from oats is purified by poly (ethylenimine) and ammonium sulfate fractionation, hydroxyapatite (HA) chromatography, and a second ammonium sulfate fractionation. Iodoacetamide is used throughout as a protease inhibitor. HA-purified phytochrome is further separated by SDS PAGE. Its location is identified by its $Zn^{2+}$-induced fluorescence. After staining with Coomassie blue, phytochrome bands are excised, stacked 7-8 at a time in the sample well of a second SDS gel, electrophoresed, transferred to nitrocellulose and visualized first by $Zn^{+2}$-induced fluorescence followed by staining with Ponceau S. Phytochrome-containing bands are excised, washed excised, washed extensively with water, dried, dissolved in dimethylsulfoxide, emulsified with Freund's adjuvant, and injected into BALB/c mice.

The purification procedure for green-plant phytochrome provided within the scope of the present invention is a novel procedure which allows an increase in scale, eliminates immunodominant contaminants and markedly improves both the purity and yield of green phytochrome. In particular, in contrast to previous attempts to purify green phytochrome, a reduction to approximately 36 % in the amount of $(NH_4)_2SO_4$ used for the initial fractionation markedly increases purity without significantly influencing yield. Further, a large reduction in the amount of buffer used to dissolve the $(NH_4)_2SO_4$ pellet does not alter yield, but does increase purity significantly, presumably by reducing the amount of contaminating proteins that are dissolved. The previous use of DEAE-agarose chromatography is eliminated here and two SDS gels are provided.

Thus, the novel purification procedure for green phytochrome as described fully in Example I below, eliminates immuno dominant proteins, as well as minimizes degradation that previously had been found to occur. The latter was important because the contaminating, immunodominant proteins are about 115-kDa in size, about the same as proteolytically degraded green phytochrome. Hydroxyapatite-purified phytochrome, prepared as described here, is free of these contaminantes. Moreover, by use of the inventive process described herein yielding green-plant phytochrome of high purity, monoclonal antibodies directed to green phytochrome can be obtained. With such electrophoretically purified phytochrome, monoclonal antibodies to contaminating proteins are never observed. Thus, it appears that the protein band detected on the second SDS gel, which correlates precisely with $Zn^{2+}$-induced fluorescence of the phytochrome chromophore, contains only phytochrome. This concentration step is essential to minimize the amount of nitrocellulose and dimethylsulfoxide injected with phytochrome during immunization.

A further embodiment of the present invention comprises a method for preparing a hybridoma cell line that produces monoclonal antibodies that react selectively with a green-plant phytochrome.

The use of hybrid somatic cell lines (hybridomas) as source of antibodies against very particular antigens derives from the work of Köhler and Milstein (1975).

The principle of the hybridoma/monoclonal antibody technology is based on the observation that when two somatic cells are fused the resulting hybrid cell has characteristic features of both parent types. In the case of monoclonal antibody production, the ability to synthesize the specific antibody derives from an immuno-competent B cell (usually a spleen cell) which has been taken from a previously immunized donor animal, whereas the ability for continuous cell division in culture is contributed by the other fusion partner, a tumour cell line (often a myeloma).

Each of these hybrid cell lines synthesizes a homogeneous immunoglobulin which represents only a single representative from the large number of possible antibodies which can be synthesized *in vivo* by an animal in response to an antigen. Since every immunoglobulin-producing clone is characterized by a single type of antibody, the name monoclonal antibodies has become established.

For preparation of immunocompetent cells donor animals are immunized by administration, one or more times, of the electrophoretically purified gree-plant phytochrome. Administration 2 or 3 times, which takes place at intervals of 7 to 40 days, but in particular 13 to 15 days, is particularly preferred.

The administration form preferred within the scope of the present invention is injection, which can take place both intravenously, intraperitoneally or subcutaneously. A combination of subcutaneous and intraperitoneal injection is preferred.

A rest period of 0.5 to 4 months is followed by another single administration of the purified gree-plant phytochrome in a dosage of 10 $\mu$g to 100 $\mu$g.

In a period of 1 to 6 days after the last dosage the donor animals are sacrificed, and a spleen cell suspension is prepared. This entails the isolated spleen cells being suspended in a suitable buffer (for example a BSS buffer) and stored in the form of a cell suspension until they are fused with suitable myeloma cells.

These fusions were initially complicated by the fact that the myeloma cell lines also synthesized monoclonal antibodies so that the hybrid produced two types of monoclonal antibodies, one having its origin in the myeloma cell and a second defined by the genetic information in the immunocompetent cell.

Hence, preferably used within the scope of the present invention are those tumour cells which are themselves unable to produce any monoclonal antibodies, such as, for example SP2/0-Ag14 (which cell line can be obtained from the American Type Culture Collection, Rockville, Maryland, USA) or X63-Ag8.653, which makes the analysis of the resulting fusion products very much more straight forward. It is advantageous for the success of fusion if the spleen cells are present in a 2 to 20-fold excess in relation to the myeloma cells. The fusion of spleen and myeloma cells is carried out in a special fusion medium which has a composition which provides optimal conditions for the intended cell fusion.

Said fusion medium is preferably a buffer solution which contains one of the fusion promoters customarily used for the fusion of cells, such as, for example, Sendai viruses or other paramyxoviruses, where appropriate in UV-inactivated form, calcium ions, surface-active lipids such as, for example, lysolecithin or polyethylene glycol. Particularly preferred within the scope of this invention is the use of polyethylene glycol (PEG), in particular of polyethylene glycol (PEG) with a mean MW of 600 to 6000, and in a concentration of 30 % to 60 %. A PEG concentration of 40 % -50 % is particularly preferred. The optimal fusion temperature is between 18°C and 39°C. A temperature of 37°C is particularly preferred.

After the fusion of the immunocompetent spleen cells with the myeloma cells has taken place the fused antibody-producing hybrid cells are selected with the aid of methods known *per se*.

In a preferred embodiment of the present invention, the fused hybrid cells are cultivated in the presence of so-called feeder cells which are macrophages isolated previously from the peritoneum of untreated non-immunized experimental animals. For the cultivation and the selection of the fused hybrid cells, the cell suspension is divided into several aliquots, and the individual aliquots are continually examined for the development of hybrid cell cultures as well as for the formation of antibodies.

Cultivation of the fused hybrid cells on microtiter plates is particularly preferred within the scope of this invention. This entails the cell suspension obtained after the fusion being distributed over the individual wells in a microtiter plate and cultivated for a period of 7 to 30 days under suitable conditions promoting the growth of the fused hybrid cells (for example HAT/HT media).

The supernatants from grown hybrid cultures are continually examined for the formation of antibodies. Positive hybrid cell cultures are then singled out using known methods, but preferably using the limiting dilution method, and subsequently cloned in suitable cultivation media.

6

The supernatants from the grown cell clones are likewise examined for the formation of antibodies. The screening of the hybridoma cell clones according to the invention, which have been prepared as described hereinbefore, for the formation of suitable monoclonal antibodies is preferably carried out using one of the immunoassays customarily used for this purpose, such as, for example, an enzyme-coupled immunoassay or a radioimmunoassay or by immunoblotting.

The present invention further relates to the preparation of monoclonal antibodies by use of methods which are known *per se* and which are characterized in that the hybridoma cell lines according to the invention, which have been charac. terized in detail previously, or else clones or subclones thereof, which synthesize, and secrete into the surrounding medium, the antibodies according to the invention, are cultivated *in vitro* or *in vivo* by known methods.

The *in vitro* cultivation of the hybridoma cells according to the invention is carried out in suitable cultivation media, in particular in the standardized culture media customarily used, such as, for example, Dulbecco's modified Eagle medium (DMEM) or RPMI 1640 medium, which can be supplemented, where appropriate, by addition of mammalian sera such as, for example, fetal calf serum, or by growth-promoting additives and trace elements.

The isolation of the monoclonal antibodies preferably starts with a precipitation of the immunoglobulin fraction from the relevant supernatants from the hybridoma cultures, for example using ammonium sulfate. This is followed by further working up and purification steps which are known to those skilled in the art and include, for example, the use of chromatographic methods such as, for example, gel filtration, ion exchange chromatography, DEAE-cellulose chromatography, protein A or immunoaffinity chromatography.

Especially preferred within the scope of the present invention is an immunoaffinity chromatography using immobilized anti-phytochrome antibodies.

Large amounts of the monoclonal antibodies according to the invention can also be obtained, however, using *in vivo* methods.

Thus, for example, it is possible to inject antibody-producing hybridoma cell clones into suitable mammals, which induce the development of antibody-producing tumours in the treated animals. It is possible after a period of 1 to 3 weeks to isolate the antibodies from the body fluids of the animals treated in this way.

In a particular embodiment of the present invention, female Balb/c mice which have, where appropriate, been pretreated with a hydrocarbon such as, for example, pristane receive intraperitoneal injection of a hybridoma cell clone according to the invention.

One to three weeks after the injection of the hybridoma cell clone the ascites fluid is collected and stored until further processed. The monoclonal antibodies are isolated in a manner exactly analogous to the isolation described previously from the supernatants from hybridomas cultivated *in vitro*.

That the new monoclonal antibodies disclosed in the present invention react selectively with a green-plant phytochrome and do not cross-react significantly with etiolated phytochrome from the same plant can be established by methods known in the art, such as, for example, immunoblotting, ELISA and/or immunoprecipitation.

As described in Example II below, hybridomas GO-4, GO-5, GO-6, GO-7, GO-8 selective for green phytochrome are produced; monoclonal antibodies are immunopurified with a column of immobilized rabbit antibodies to mouse immunoglobulins.

A futher embodiment of the present invention comprises a method for purifying a green-plant phytochrome suitable for use as an immunogen to produce hybridomas that react selectively with a green-plant phytochrome comprising purifying a green-plant phytochrome by immunoaffinity chromatography utilizing monoclonal antibodies produced by at least one hybridoma of the present invention. Expecially preferred within the scope of the present invention is a monoclonal antibody produced by the hybridoma GO-5.

A partial amino acid sequence of the immunoaffinity-purified green-plant phytochrome can be obtained by methods known in the art. Preferred within the present invention is performing an automated Edman degradation with a gas-phase sequencer and identifying the resulting phenylthiohydantoin amino acids using an on-line Analyzer.

Protein sequence analysis of immunoaffinity-purified green-plant phytochrome yieldes a partial amino acid sequence
V-A-E-I-T-G-L-P-T-M-E-A-I-G-M-P-L-V-D
which when compared with the known amino acid sequences for etiolated phytochrome demonstrates for the first time that green-plant phytochrome is encoded by a separate gene (or genes) than that which encodes for the amino acid sequence of etiolated phytochrome.

The sequence of amino acid residues is designated herein either through the use of their commonly employed singleletter designations. A listing of these one-letter and the three-letter designations may be found in textbooks such as Biochemistrv, Lehninger A, Orth Publishers, New York, NY (1970). When the amino acid sequence is listed horizontally, the amino terminus is inteded to be on the left end whereas the carboxy terminus is intended to be at the right end.

Using the amino acid sequence information, the DNA sequences capable of encoding them are examined in order to construct oligonucleotide probes for identifying the gene sequences encoding the green-plant phytochrome. Because the genetic code is degenerate, more than one codon may be used to encode a particular amino acid.

Although occasionally an amino acid sequene may be encoded by only a single oligonucleotide, frequently the amino acid sequence may be encoded by any of a set of similar oligonucleotids. Importantly, whereas all of the members of this set contain oligonucleotides which are capable of encoding the peptide fragment and, thus, potentially contain the same oligonucleotide sequence as the gene which encodes the peptide fragment, only one member of the set contains the oligonucleotide sequence that is identical to the nucleotide sequence of the gene. Because this member is present within the set, and is capable of hybridizing to DNA even in the presence of the other members of the set, it is possible to employ the unfractionated set of oligonucleotides in the same manner in which one would employ a single oligonucleotide to clone the gene that encodes the peptide.

Using the genetic code, one or more different oligonucleotides can be identified, each of which would be capable of encoding the above partial amino acid sequence of green-plant phytochrome peptides. The probability that a particular oligonucleotide will, in fact, constitute the actual encoding sequence contained in the green-plant phytochrome gene(s) can be estimated by considering abnormal base pairing relationship and the frequency with which a particular codon is actually used (to encode a particular amino acid) in prokaryotic or eukaryotic cells. Such "codon usage rules" are disclosed by Lathe R et al (1985). Using the "codon usage rules", a single oligonucleotide, or a set of oligonucleotides, that contains a theoretical "most probable" nucleotide sequence capable of encoding the green-plant phytochrome partial peptide sequence can be identified.

The oligonucleotide, or set of oligonucleotides, containing the theoretical "most probable" sequence capable of encoding the green-plant phytochrome gene fragments is used to identify the sequence of a complementary oligonucleotide or set of oligonucleotides which is capable of hybridizing to the "most probable" sequence, or set of sequences. An oligonucleotide containing such a complementary sequence can be employed as a probe to identify and isolate the green-plant phytochrome gene [Maniatis T et al - (1982)].

Thus, in summary, the actual identification of a partial green-plant phytochrome peptide sequences permits the identification of a theoretical "most probable" DNA sequence, or a set of such sequences, capable of encoding such a partial peptide. By constructing an oligonucleotide complementary to this theoretical sequence (or by constructing a set of oligonucleotides complementaryto the set of "most probable" oligonucleotides), one obtains a DNA molecule (or set of DNA molecules), capable of functioning as a probe to identify and isolate the green-plant phytochrome gene.

From the sequence of amino acids of a partial green-plant phytochrome peptide and the above consideration, the following oligonucleotide with permissible base substitutions noted underneath at the appropriate sites can be designed:

```
CCA ACA ATG GAG GCA ATT GGA ATG CC
..T ..T     ..A ..T ..C ..T
..C ..C         ..C ..A ..G
..G ..G         ..G     ..C
```

A preferred oligonucleotide with permissible base substitutions noted underneath at the appropriate sites is as follows:

```
CCA ACA ATG GAG GCA ATT GGA ATG CC
..T ..T     ..A ..T ..C ..T
    ..C             ..G
                    ..C
```

8

The said oligonucleotide probes may be used in standard methods known and used by those of skill in the art to clone and isolate green-plant phytochrome promoters and structural genes from plant genomic libraries and to clone and isolate green-phytochrome cDNAS from plant cDNA libraries.

Genomic DNA or cDNA may be obtained in several ways. Genomic DNA can be extracted and purified from suitable cells by means well known in the art. Alternatively, mRNA can be isolated from a cell which produces the green-plant phytochrome and used to produce cDNA by means well known in the art. Such suitable DNA preparations are enzymatically cleaved, or randomly sheared, and ligated into cloning vectors to form a gene library. Such vectors can then be screened with the above-described oligonucleotide probes in order to identify a green-plant phytochrome encoding sequence.

A suitable oligonucleotide, or set of oligonucleotides, which is capable of encoding a fragment of the green-plant phytochrome gene (or which is complementary to such an oligonucleotide, or set of oligonucleotides) identified using the above-described procedure, is synthesized, and hybridized by means well known in the art, against a DNA or, more preferably, a cDNA preparation derived from cells which are capable of expressing the green-plant phytochrome gene. The source of DNA or cDNA used will preferably have been enriched for green-plant phytochrome sequences. Such enrichment can most easily be obtained from cDNA obtained by extracting RNA from cells which produce high levels of the green-plant phytochrome gene. Techniques of nucleic acid hybridization are disclosed by Maniatis T *et al* (1982), and by Haymes BD *et al* (1985), which references are herein incorporated by reference.

To facilitate the detection of the desired green-plant phytochrome encoding sequence, the above-described DNA probe may be labeled with a detectable group. Such detectable group can be any material having a detectable physical or chemical property. Such materials have been well-developed in the field of immunoassays and in general any label useful in such methods can in principal be applied to the present invention. Particularly useful are enzymatically active groups, such as enzymes, enzyme substrates, coenzymes and enzyme inhibitors; fluroescers; chromophores; luminescers such as chemiluminescers and bioluminescers; specifically bindable ligands; proximal interacting pairs; and radioisotopes such as $^3H$, $^{35}S$, $^{32}P$, $^{125}I$ and $^{14}C$. Such labels and labeling pairs are detected on the basis of their own physical properties (e.g., fluorescers, chromophores and radioisotopes) or their reactive or binding properties (e.g. enzymes, substrates, coenzymes and inhibitors). For example, a cofactor-labeled probe can be detected by adding the enzyme for which the label is a cofactor and a substrate for the enzyme. For example, one can use an enzyme which acts upton a substrate to generate a product with a measurable physical property. Examples of the latter inlcude, but are not limited to, beta-galactosidase, alkaline phosphatase and peroxidase.

General procedures for hybridization are disclosed, for example, in Maniatis T (1982) and in Haymes BT *et al* (1985). Those members of the above-described gene library which are found to be capable of such hybridization are then analyzed to determine the extent and nature of the green-plant phytochrome encoding sequences which they contain.

In an alternative way of cloning the green-plant phytochrome gene, a library of expression vectors is prepared by cloning DNA or, more preferably cDNA, from a cell capable of expressing green-plant phytochrome into an expression vector. The library is then screened for members capable of expressing a protein which binds to an anti-green-plant phytochrome antibody, and which has a nucleotide sequence that is capable of encoding polypeptides that have the same amino acid sequence as the green-plant phytochrome or fragments of the green-plant phytochrome.

The cloned green-plant phytochrome encoding sequences, obtained through the methods described above, may be operably linked to an expression vector, and introduced into bacterial, or eukaryotic cells to produce green-plant phytochrome or a functional derivative thereof. Techniques for such manipulations are disclosed by Maniatis T *et al* (1982), and are well known in the art.

In Example III below, the immunoaffinity purification of green phytochrome using GO-5 monoclonal antibodies is described. Protein sequence analysis of immunoaffinity-purified green phytochrome yieldes a partial amino acid sequence which when compared with the known amino acid sequence for etiolated phytochrome demonstrates for the first time that green phytochrome is encoded by a separate gene (or genes) than that which encodes for the amino acid sequence of etiolated phytochrome.

From the partial amino acid sequence for green phytochrome, oligonucleotide probes are designed which may be used in standard methods known and used by those of skill in the art to clone and isolate green-phytochrome promoters and structural genes from plant genomic libraries and to clone and isolate green-phytochrome cDNAS from plant cDNA libraries. Example IV describes the procedures to be used to clone green-phytochrome genes.

The invention described herein has many important applications. Monoclonal antibodies that are specific for green-plant phytochrome can be used to quantitate green-plant phytochrome. Further, green-plant phytochrome is important in a plant growing in a natural environment, such as an agricultural field, and influences crop yields in many plants.

Thus, the isolation of green-plant phytochrome genes is essential for the eventual genetic manipulation of plants to increase plant productivity in a natural environment.

Deposition

On October 27, 1988, the following three hybridoma cell lines were deposited with the American Type Culture Collection:

GO-5 14.9.88 JJB
ATCC HB 9885
GO-7 15.9.88 JJB
ATCC HB 9886
GO-8 15.9.88 JJB
ATCC HB 9887

Non-limiting Working Examples

Example I - Purification of Green Phytochrome

This purification procedure for green-plant phytochrome is a novel procedure which allows an increase in scale, eliminates immunodominant contaminants and markedly improves both the purity and yield of green phytochrome. In particular, in contrast to previous attempts to purify green phytochrome, a reduction to 36 % in the amount of $(NH_4)_2SO_4$ used for the initial fractionation markedly increases purity without significantly influencing yield. Further, a large reduction in the amount of buffer used to dissolve the $(NH_4)_2SO_4$ pellet does not alter yield, but does increase purity significantly, presumably by reducing the amount of contaminating proteins that are dissolved. The previous use of DEAE-agarose chromatography is eliminated here and two SDS gels are provided.

A) Plant material.

Shoots are harvested from etiolated oats (*Avena sativa* L. cv. Garry) grown for 5 days at 25° C in total darkness and high humidity. See Pratt LH (1973), incorporated herein by reference. Green oats are grown for a period of 10 to 11 days under natural illumination in a greenhouse. Leaves are harvested in daylight, just before sunset. Harvested tissue is immediately frozen and stored at -20° C until used.

B) Spectrophotometric assay.

Phytochrome is measured by photoreversibility assay at 664 nm and 728 nm for etiolated phytochrome, and at 653 nm and 720 nm or 728 nm for green phytochrome, using a fully automated, custom-built, dual-wave-length spectrophotometer similar in principle to that described by Pratt LH et al, (1985). Phytochrome quantities from etiolated oats are calculated from $\Delta\Delta A$ units [Pratt LH, (1983)] using extinction coefficients obtained by Litts JC et al, (1983). Green phytochrome amounts are estimated similarly by assuming that it has the same extinction coefficients as does etiolated phytochrome. In both cases, one $\Delta\Delta A$ unit is about 1 mg of phytochrome. One unit (U) of phytochrome is the quantity that gives, in a volume of 1 ml and for a light path of 1 cm, a $\Delta\Delta A$ of 1.0 at the above wavelengths. Immunoprecipitates and resultant supernatants are assayed after addition of $CaCO_3$ as a scattering agent (500 mg/ 400 $\mu$1 sample), which increases the effective optical light path [Butler WL, (1962)].

Absorbance spectra of hydroxyapatite-purified preparations of phytochrome are obtained with a split-beam diode-array spectrophotometer (Hewlett Packard 8452A) interfaced with a PC's Limited 286[8] micro-computer (Dell Computer Corp., Austin, TX) and operated with custom-written software [Wampler JE et al, (1979); Rich ES Jr. and Wampler JE, (1982)]. The sample cuvette is cooled with circulating ice water.

C) Phytochrome purification.

All work with phytochrome is done under green light with phytochrome maintained at or below 2° C, unless otherwise specified, or until phytochrome is denatured with SDS sample buffer.

For purification of green phytochrome, frozen green oat shoots, chopped into 3-4 cm long pieces with a sharp razor blade, are extracted in 500 g lots with 375 ml of extraction buffer in a 4 l Waring Blender. The extraction buffer, which consists of 0.1 M tris-HCl, 0.14 M $(NH_4)_2SO_4$, 10 mM EDTA and 50 % ethyleneglycol, is chilled to -20° C before use. Iodo. acetamide is freshly added, as a protease inhibitor, from a 0.2 M stock solution to a concentration of 18 mM in the extraction buffer and 10 mM in all subsequent buffers. In addition, 0.87 g of sodium bisulfate is added immediately prior to homogenization of the tissue. As soon as the tissue is thoroughly extracted, it was filtered rapidly by centrifugation through a layer of Miracloth in a Braun MP50 juice extractor. Ten milliliter of 10 % (v/v) poly(ethylenimine) is added per liter of filtrate, which is then mixed for 1 minute and centrifuged for 15 minutes at 25,000 g. $(NH_4)_2SO_4$ at 95 % saturation and containing 10 mM iodoacetamide at pH 7.8 is added to the supernatant to a final concentration of 36 %. After stirring for 1 minute, the preparation is centrifuged for 15 minutes at 25,000 g. The pellet is dissolved in 25 ml of resuspension buffer (50 mM tris-HCl, 5 mM EDTA, 10 mM iodoacetamide, 25 % (v/v) ethylene glycol, pH 7.8 at 4° C), clarified, frozen in liquid $N_2$, and stored at -80° C. Eight such preparations are accumulated for further purification by hydroxyapatite chromatography.

The eight preparations are thawed, clarified by centrifugation for 10 minutes at 50,000 g, and diluted to a total volume of 1360 ml with resuspension buffer. The combined sample is passed through a 200 ml bed-volume hydroxyapatite column (Pratt LH, In: Techniques in Photomorphogenesis, Edited by Smith H and Holmes MG, pp. 175-200, Academic Press, London, New York, 1984) equilibrated with resuspension buffer containing 70 mM $(NH_4)_2SO_4$. The column is washed first with 400 ml of 50 mM tris-HCl, 5 mM EDTA, 10 mM iodoacetamide, pH 7.8 at 4° C, then with 400 ml of 5 mM K-phosphate, 5 mM EDTA, 10 mM iodoacetamide, pH 7.8. Phytochrome is eluted with a linear gradient prepared from 600 ml each of 5 mM and 200 mM K-phosphate, each containing 5 mM EDTA and 10 mM iodoacetamide at pH 7.8. The column is operated throughout at 8 ml/min. The peak fractions are kept as pool I, while the adjacent fractions on either side of the peak are kept as pool II) see Table 1 below). Phytochrome is precipitated by addition of 95 % saturation $(NH_4)_2SO_4$ containing 10 mM iodoacetamide at pH 7.8 to a final concentration of 30 %, collected by centrifugation for 15 min at 40,000 g, redissolved in 0.1 M Na-phosphate, 1 mM EDTA, pH 7.8, frozen in liquid $N_2$, and stored at -80° C.

Table 1.

| | Volume (ml) | Phytochrome | | Protein[1] | | Purity[2] | Yield % |
|---|---|---|---|---|---|---|---|
| Summary of green-oat phytochrome content, purity and yield during purification. Entries are averages of five sequential purifications, each from 4 kg of green oat leaves. | | | | | | | |
| | | mU/ml | mU | mg/ml | mg | | |
| First (NH₄)₂SO₄ cut | | | | | | | |
| Before dilution[3] | 220 | 5.75 | 1265 | 5.40 | 1190 | 1.07 | 100 |
| After dilution | 1360 | 0.70 | 952 | 0.92 | 1251 | 0.76 | 75 |
| Hydroxyapatite | | | | | | | |
| Pool I | 89 | 4.53 | 403 | 1.45 | 129 | 3.1 | 32 |
| Pool II | 66 | 2.97 | 196 | 1.42 | 94 | 2.1 | 15 |
| Second (NH₄)₂SO₄ cut | | | | | | | |
| Pool I | 3.8 | 107 | 407 | 18.3 | 70 | 5.8 | 32 |
| Pool II | 3.1 | 56 | 174 | 19.7 | 61 | 2.8 | 14 |

[1] Protein content estimated by Bradford assay (Bradford, 1976), with reference to bovine serum albumin.

[2] Phytochrome purity is expressed as mU/ml phytochrome divided by mg/ml protein

[3] Sum of eight 500 g preparations after resuspension and clarification, but before freezing.

D) Electrophoresis and electroblotting.

SDS-polyacrylamide gel electrophoresis [Laemmli UK, (1970)] and electroblotting [Towbin H *et al*, (1979)] are done as described in Pratt LH *et al* [cited in: Linskens HF and Jackson JF (1986)], incorporated herein by reference. When phytochrome is visualized in a gel by $Zn^{2+}$-inducedchromophore fluorescence under UV light, $Zn^{2+}$ is included both in the gel and running buffers as described by Berkelman T and Lagarias JC, (1986). When $Zn^{2+}$-induced phytochrome fluorescence is visualized on nitrocellulose, 1 mM $Zn^{2+}$ is also added to the electrotransfer buffer. Molecular weight standards are obtained from Sigma (SDS-6H).

E) Phytochrome preparation for immunization.

Samples of 30 $\mu$1 of hydroxyapatite-purified green-oat phytochrome (about 1 $\mu$g phytochrome) prepared in SDS sample buffer are added to each of 20 lanes of a 5% to 10 % linear-gradient, SDS polyacrylamide gel (11 cm x 14 cm) (Laemmli, 1970). After electrophoresis, the position of phytochrome in preliminary gels is visualized by its $Zn^{2+}$-induced fluorescence [Berkelman T and Langaria JC, (1986)], in which case the positions of the fluorescent bands are marked by punching holes in the gel. The gel is then stained with Coomassie blue and phytochrome-containing bands were excised. The excised bands are boiled in SDS sample buffer, placed 7 at a time into each lane of a 6 %, 10-lane, SDS polyacrylamide gel (ca. 6 cm x 14 cm), and re-electrophoresed. This second gel is short, having a stacking gel that is about half its total length. The re-electrophoresed phytochrome is electrotransferred to nitrocellulose, on which it is again visualized by $Zn^{2+}$-induced fluorescence in initial preparations, as well as by Ponceau S stain. When $Zn^{2+}$-induced fluorescence is observed, the fluorescent bands are marked by outlining them with a pencil. The portion of the nitrocellulose bearing phytochrome is excised, washed exhaustively with water, and air dried for storage at room temperature.

F) Results

Modification of the previously used procedure for purification of phytochrome [Shimazaki Y and Pratt LH , (1985)] improves markedly both its purity and yield (Table 1). Based on extinction coefficients published by Lagarias JC, et al., (1987), and on the assumption that phytochrome from both etiolated and green oat shoots have the same extinction coefficients, the hydroxyapatite-purified phytochrome obtained here is about 0.5 % pure, which represents an estimated 250-fold purification.

Green-oat phytochrome samples prepared as described herein, are at least 10-fold more concentrated, more free of contaminating pigmentation, and significantly purer than those described previously [Tokuhisa JG, et.al. (1985); Shimazaki Y and Pratt LH (1985); Cordonnier M-M et al (1986)]. By including iodacetamide as a protease inhibitor, phytochrome is obtained which exhibited on immunoblots predominantly, although not exclusively with all monoclonal antibodies, a single immunostained band near 124 kDa. This band co-migrates with the green-oat phytochrome that is immunostained when SDS sample buffer extracts of lyophilized, green oat leaves are immunoblotted [see Cordonnier M-M et al (1983)].

The purification procedure for green phytochrome described herein eliminates immunodominant proteins, as well as minimizes degradation that previously had been found to occur. The latter is important because the contaminating proteins are about 115-kDa in size, about the same as proteolytically degraded green phytochrome. Hydroxyapatite-purified phytochrome, prepared as described here, is free of these contaminantes. Moreover, by cutting out only the Coomassie blue-stained band immediately above the 116-kDa $\beta$-galactosidase marker while ignoring that just below it, monoclonal antibodies directed to green phytochrome can be obtained. With such electrophoretically purified phytochrome, monoclonal antibodies to contaminating proteins are never observed. Thus, it appears that the protein band detected on the second SDS gel, which correlated precisely with $Zn^{2+}$-induced fluorescence of the phytochrome chromophore, contains only phytochrome. This concentration step is essential to minimize the amount of nitrocellulose and dimethylsulfoxide injected with phytochrome during immunization.

Example II - Production of Hybridoma Cell Lines

Five new hybridoma cell lines GO-4, GO-5, GO-6, GO-7, GO-8 secreting monoclonal antibodies directed to green-oat phytochrome are produced by the following method.

A) Hybridomas and Antibodies.

Female BALB/c mice are immunized with dimethylsulfoxide-dissolved nitrocellulose (50 $\mu l/cm^2$) that contained electrophoretically purified green-oat phytochrome and that is emulsified with an equal volume of complete Freund's adjuvant. An estimated 32 $\mu$g to 40 $\mu$g of phytochrome is injected. Mice are similarly re-injected one month after the first injection with the same amount of phytochrome, then again with the same amount 14 days later, except that incomplete Freund's adjuvant is used. Three days after the last injection, spleens are harvested and fusions performed as described in Cordonnier M-M et al (1983), incorporated herein by reference. Fusion products are screened both by immunoblotting and by an amplified ELISA. Positive cell lines are subcloned and expanded for antibody production, as well as preserved by freezing and cryogenic storage.

Antibodies are immunopurified as described in Cordonnier M-M et al (1983). Oat-3, Oat-9, Oat-22 and Pea-25 have been characterized previously [e.g., Shimazaki and Pratt (1985); Cordonnier M-M et al (1986)]. Polyclonal rabbit antibodies to phytochrome are directed to ca. 120 kDa etiolated-oat phytochrome. Antiphytochrome immunoglobulins are purified by adsorption to and elution from a column of immobilized, ca. 120-kDa etiolated-oat phytochrome [Pratt LH, cited in: Smith H and Holmes MG (1984)].

B) Immunoblotting.

Electroblots of SDS polyacrylamide gels are immunostained as described in detail in Pratt LH et al - [cited in: Linskens HF and Jackson JF (1986)], incorporated herein by reference. The protocol utilizes mouse monoclonal antibody followed by rabbit antibodies to mouse immuno globulins and alkaline phosphates-conjugated goat antibodies to rabbit immunoglobulins, or rabbit antibodies followed by goat antibodies to rabbit immunoglobulins and alkaline phos phatase-conjugated rabbit antibodies to goat immunoglobulins. For screening of hybridomas, electroblots of 15-lane, 6 % minigels (5.5 cm x 8 cm) are prepared, with 50 ng green-oat phytochrome per lane. The region of each lane expected to contain phytochrome is cut out and placed in a well of a 48-well culture plate. Otherwise, the immunostaining protocol is as for a normal immunoblot.

## C) ELISAs.

Details about the procedures used to perform ELISAs, as well as buffer compositions, may be found in Pratt LH *et al* [cited in: Linskens HF and Jackson JF (1986)]. The screening ELISA is as in Cordonnier, M-M, *et al* (1983), with the following modifications. (i) Wells are coated with 1μg/ml of hydroxyapatite-purified green-oat phytochrome. (ii) The number of washes between each incubation with antibody is increased from three to five or more to reduce background. (iii) The alkaline phosphatase-conjugated rabbit antibodies to mouse immunoglobulins are diluted 1000-fold instead of 500-fold. (iv) A modified substrate that amplifies the alkaline phospatase activity is used. [Johannson A *et al*, (1986)].

Color development occurrs in two steps. First, 50 $\mu$1 of 100 $\mu$M NADP (Sigma N-0505) in 50 mM diethanolamine, 1 mM $MgCl_2$, pH adjusted to 9.5 with HCl, is added to each well. After incubation for 30 min at room temperature, 100 $\mu$l of a second substrate solution is added to the first. The second substrate solution is prepared by mixing together (i) 4.8 ml of 25 mM Na-phosphate, pH 7.2, (ii) 1.2 ml of 2.75 mM tetrazolium salt (INT, Sigma I-8377) in 25 mM Na-phosphate, 20 % (v/v) ethanol, pH 7.2, (iii) 15 $\mu$1 of 5 mg/ml alcohol dehydrogenase (Sigma A-3263) in 50 mM tris-HCl, 40 % (v/v) glycerol, 0.1 % $NaN_3$. 10 mg/ml bovine serum albumin, pH 8.0 at 25$^\circ$C, and (iv) 150 $\mu$l of 5 mg/ml diaphorase (Sigma D-2381) in 25 mM Na-phosphate, pH 7.2. The alcohol dehydrogenase stock solution is stored at 4$^\circ$C to prevent loss of enzyme activity that occurrs upon freezing and thawing; other stock solutions were stored in small aliquots at -20$^\circ$C. After incubation for 30 minutes at room temperatures, or until sufficient purple color has developed, the reaction is stopped by the addition of 50 $\mu$l of 0.4 N HCl. The ELISA plate is assayed at 500 nm as quickly as possible, since the reaction product precipitates with time.

## D) Immunoprecipitations.

The general protocol used here is identical to that described in Shimazaki Y and Pratt LH, (1985); Cordonnier M-M *et al* (1986), except for the specific ratios of antibody to phytochrome used. An immunoprecipitate of etiolated-oat phytochrome is prepared by mixing 16.5 μg of hydroxyapatite-purified etiolated-oat phytochrome with 3.3 μg of monoclonal antibody Oat-3, which is equivalent to 3 phytochrome monomers per antigen-binding site. *Staphylococcus aureus* cells (Pansorbin; Calbiochem-Behring, La Jolla, CA, USA) are used as before to precipitate the monoclonal antibody with bound phytochrome. The amount of phytochrome in the pellet is estimated from the amount remaining in the supernatant, as assayed spectrophotometrically.

Immunoprecipitates of green-oat phytochrome are prepared for two purposes. In order to determine whether a given mono clonal antibody can immunoprecipitate phytochrome photoreversibility (see Table 2 below), and show that precipitated product is detected by Pea-25 monoclonal antibodies, 2 mU of phytochrome were incubated with the indicated amount of monoclonal antibody. After further incubation with 180 $\mu$l of a 10 % suspension of *Staphylococcus* aureus cells and centrifugation, both pellets and supernatants are assayed for photoreversibility. Because $CaCO_3$ was used, the difference in light scattering between these two samples is essentially nullified. The second purpose is to characterize the precipitated green phytochrome.

Table 2.

| Immunoprecipitation of green-oat phytochrome photo-reversibility. | | | |
|---|---|---|---|
| Mab | Amount (μg) | Phytochrome | |
| | | Pellet | Supernatant |
| GO-4 | 5 | 29 | 71 |
| GO-5 | 5 | 29 | 71 |
| GO-6 | 2 | 29 | 71 |
| GO-7 | 70 | 19 | 81 |
| GO-8 | 20 | 28 | 71 |
| Oat-9 | 20 | 21 | 79 |

Two milliunits of green-oat phytochrome are mixed with the indicated quantity of monoclonal antibody. After incubation with 180 μl of *Staphylococcus aureus* and centrifugation, both pellets and supernatants are assayed for photoreversibility. Results are normalized to 100 % for the sum in each case. Phytochrome amounts in the pellets of non-immune mouse immunoglobulin controls (2 μg to 70 μg) ranged from 1% to 3 %.

For preparation of immunoblots of immunoprecipitates, 4 mU of green-oat phytochrome is incubated with the following monoclonal antibodies in the indicated amounts: GO-4, 10 μg; GO-5, 10 μg; GO-6, 10 μg; GO-7, 50 μg; GO-8, 20 μg. After incubation for 2 h at 2°C, 180 μl of a 10 % suspension of *Staphylococcus aureus* cells is added as before and the cells are collected by centrifugation (Shimazaki and Pratt, 1985). Pellets are washed two times in 0.05 % Triton X-100, 10 mM tris-HCl, 0.15 M NaCl, pH 8.0. The amount of phytochrome present in each pellet is estimated from photoreversibility assays of the corresponding supernatants.

To prepare immunoblots, double strength sample buffer is added to pellets to a final estimated phytochrome concentration of 4 μg/ml. Samples are incubated at 100°C for 2 min and are loaded onto a 5% to 10 % gradient SDS polyacrylamide gel.

E) Results

Five new hybrid cell lines designated GO-4, GO-5, GO-6, GO-7 and GO-8 are produced which secrete monoclonal antibodies specific for green phytochrome.

Each monoclonal antibody detects a polypeptide of the same size as that stained by PEA-25 on immunoblot, shown earlier to cross react with green-oat phytochrome [Cordonnier *et al*, (1986)], regardless of the extend of degradation of phytochrome. Except for Pea-2 and Oat-15, which recognize the same epitope as Pea-25 on immunoblot, both polyclonal antibodies (RAP) and monoclonal antibodies (Oat-13, Oat-22) to etiolated-oat phytochrome detect green-oat phytochrome either weakly or not at all. Conversely, these new monoclonal antibodies to green-oat phytochrome detect etiolated-oat phytochrome poorly, if at all, on immunoblot.

Each of the new monoclonal antibodies immunoprecipitates phytochrome-associated photoreversibility from hydroxyapatite-purified green-oat phytochrome preparations. As before [Shimazaki and Pratt, (1985)], Oat-9 also immunoprecipitates photoreversilibity from green-oat phytochrome preparations. The polypeptide immunoprecipitated by each of the monoclonal antibodies directed to green-oat phytochrome is immunostained well with Pea-25, but not Oat-22.

Monoclonal antibody Oat-3, which was shown previously not to immunoprecipitate green-oat phytochrome [Shimazaki and Pratt, (1985)], is used to precipitate hydroxyapatite-purified etiolated-oat phytochrome. While Oat-22 detects this immuno precipitated phytochrome well on an immunoblot, as expected, the new monoclonal antibodies to green-oat phytochrome does so only poorly, if at all, even at high protein loads.

Demonstration that monoclonal antibodies are directed to green-oat phytochrome

That the five new monoclonal antibodies described here are unequivocally directed to green-oat phytochrome is established by two observations. First, they each immuno-precipitate phytochrome photoreversibility, although in variable amounts (Table 2). Second, the precipitation product in each case is recognized by Pea-25, a monoclonal antibody directed to etiolated phytochrome that has previously been shown to cross-react specifically by immunoblot with green-oat phytochrome in crude extracts of green oat shoots [Cordonnier et al, (1986)]. Moreover, each of the new monoclonal antibodies detects the size decrease that accompanies partial proteolytic degradation of green-oat phytochrome, a size change not seen with the apparently immunodominant contaminants referred to above.

Cross reaction of monoclonal antibodies to green-oat phytochrome with etiolated-oat phytochrome

Oat-22 has been shown previously to be specific to etiolated oat, as compared to green-oat phytochrome [Cordonnier et al, (1986)]. Its inability to cross-react by immunoblot with the precipitation products of monoclonal antibodies to green-oat phytochrome verifies that the photoreversible phytochrome that these monoclonal antibodies immunoprecipitate is different from etiolated-oat phytochrome.

To investigate further the selectivity of these new monoclonal antibodies for green-oat phytochrome, they are tested against the immunoprecipitate obtained by incubation of Oat-3 with hydroxyapatite-purified etiolated-oat phytochrome. Oat-3 was selected because of its inability to immunoprecipitate phytochrome from green oat leaves [Shimazaki and Pratt, (1985)]. Moreover, the immunoprecipitate is made with an excess of phytochrome (3 phytochrome monomers per antigen binding site) such that any weak interaction between Oat-3 and green-oat phytochrome would be unlikely to result in direct immunoprecipitation of the immunochemically distinct phytochrome present in a green-oat phytochrome preparation. Under these conditions, monoclonal antibodies directed to green-oat phytochrome do not cross react visibly with the phytochrome that was immunoprecipitated when 10 ng of phytochrome are loaded per gel, even though Oat-3 detects it readily.

Example III - Partial Amino Acid Sequence of Green Phytochrome and Synthetic Oligonucleotide For Identifying Green Phytochrome Genes

A) Immunoaffinity purification of green-oat phytochrome using GO-5 Monoclonal Antibodies.

Hydroxyapatite-purified green-oat phytochrome is purified on a immunoaffinity column of GO-5 monoclonal antibodies immobilized on affi-gel 10 beads (Biorad; Richmond, Ca.). The column constitutes 1 mg of GO-5 monoclonal antibodies on 1 ml of packed affi-gel 10 beads.

The column is equilibrated with 25 mM Tris, 140 mM NaCl, pH 7.8 at 4°C prior to loading approximately 250 μg of photo-reversible phytochrome. The sample is reapplied several times until phytochrome is maximally bound as determined by spectrophotometric assay. The column is then washed with 1 ml of equilibration buffer containing 1 M NaCl. A volume of 3 ml of equilibration buffer is added. All wash solutions are spectrophotometrically monitored for phytochrome loss. A final rinse with 0.02 M Tris, pH 8.0 is performed immediately prior to elution. Phytochrome is eluted with 1 ml formic acid. Residual liquid is expelled by forcing air through the column until dry. This sample is immediately reduced to a volume of approximately 75 μl, which can be achieved, for example, by placing the sample on a Savant speed vac concentrator, and then ddH$_2$O is added to a final volume of 500 μl. This wash procedure is repeated 3 times. For the final wash, volume is reduced to approximately 150μl. This final sample is then run on 10% to 15 % Pharmacia Phastgels and stained with Coomassie R-250 to confirm purity and estimate quantity against a standard curve of beta-glactosidase (Sigma G-8511).

B) Protein Sequence Analysis of Immunoaffinity Purified Green-Oat Phytochrome

1) Peptide generation:

Tris-HCl is added to 60 μg of immuno. affinity purified phytochrome sample received in water to a concentration of 100 mM. The protein is reduced using volatile tributylphosphine at a concentration of 0.05 % under nitrogen for 30 minutes at room temprature. Following reduction, tributylphosphine is removed under vacuum ( for example, in a Savant Speed-Vac for 15 minuntes). No further modification of cysteines is attempted. Peptides are prepared by digestion with Lys-C (Boehringer-Mannheim) by adding enzyme to 2.0 % for 24 hours at room temperature.Peptides are separated by reverse phase HPLC on a 1 x 250 mm Aquapore PR-300 column in 0.1 % trifluoracetic acid using a gradient of 0% to 80 % isopropanol:acetronitrile (1:1).

2) Amino acid sequence analysis:

Automated Edman degradations are perfomred with an Applied Biosystems 470A gas-phase sequencer. Phenylthiohydantoin amino acids are identified using the on-line Applied Biosystems 120A PTH Analyzer.

3) Amino acid analysis:

Amino acid composition is determined once on a sample of approximately 3 $\mu$g using the protocol of Bidlingmeyer *et al* (1984). Gas phase hydrolysis with 6 N HCl is carried out for24 hours at 108 $^\circ$C. The phenylthiocarbamyl amino acid derivatives are analyzed by reverse phase HPLC on a Hewlett-Packard 1090 Liquid Chromatograph using an Applied Biosystems PTC-C18 column. The column is equilibrated in 3.5 mM sodium acetate, pH 5.4 and eluted with a stepwise gradient from 7 % to 60 % acetonitrile. The number of residues of each amino acid is calculated using a molecular weight of 124,000. Tryptophan and cysteine content is not determined.

4) Amino acid sequence:

A partial amino acid sequence is obtained for green-oat phytochrome:
V-A-E-I-T-G-L-P-T-M-E-A-I-G-M-P-L-V-D.
The green-oat phytochrome peptide sequenced has homology with both etiolated-zucchini and etiolated-oat phytochrome.

```
Etiolated-zucchini phytochrome 647 IAELTGLPVDKAIGKHLLTLVE 669
Green-oat phytochrome             ? VAEITGLPTMEAIG...MPLVD ?
Etiolated-oat phytochrome       649 AAELTGLRVDDAIGRHILTLVE 671
                                    uiiciiiccuciii...cciic
                                    1   5    10      15  19
```

The small letters indicate which residues are identical (i), evolutionarily conserved (c) or unrelated (u).

5) Oligonucleotides for identifying green-phytochrome gene sequences:

From the sequence of amino acids and considerations known to those of skill in the art, the following oligonucleotide with permissible base substitutions noted underneath at the appropriate sites is designed:

```
CCA ACA ATG GAG GCA ATT GGA ATG CC
..T ..T        ..A ..T ..C ..T
..C ..C            ..C ..A ..G
..G ..G            ..G     ..C
```

A preferred oligonucleotide with permissible base substitutions noted underneath at the appropriate sites is as follows:

```
CCA ACA ATG GAG GCA ATT GGA ATG CC
..T ..T        ..A ..T ..C ..T
    ..C                    ..G
                           ..C
```

Example IV - Cloning of Green-Plant Phytochrome Genes with Green-Phytochrome - specific Oligonucleotide Probes

A) Synthesis and 5′ End Labelling of oligonucleotides to be used as probes for screening of libraries.

For green phytochrome a mixture of 26-base oligonucleotides with the sequences

```
CCAACAATGGAGGCAATTGGAATGCC
    T T     A T C T
    C               G
                    C
```

and a second mixture of 26-base oligonucleotides with the sequences

```
GGCATTCCAATTGCCTCCATTGTTGG
    A  G A T    A A
    C            G
    G
```

are synthesized, used random base insertion on an Applied Biosystem Synthesizer and B-cyanoethylphosphoramidite chemistry. The oligonucleotides are purified by reverse-phase high pressure liquid chromatography (HPLC). 5 pmoles of the oligonucleotide mixture are kinased [Maniatis T et al (1982)] using 200 μC of 32P-ATP (6000 Ci/mMole, 10 μCi/μl) and T4 polynucleotide kinase. After incubation at 37°C for 30 minutes, the reaction is diluted to 100 μl, phenol/chlorform extracted and then precipitated with ethanol. The specific activity of the labeled oligonucleotide mixture is determined to be about 3 x 10⁶ Cvcpm/pMole.

B) Production of Oat Libraries and Subclones

1) Nuclei are isolated from 4 day-old etiolated oat plants *Avena sativa* L. cv Garry by first freezing 35 grams of the tissue in liquid nitrogen and grinding to a fine powder with a mortar and pestle. The powder is added to 250 ml of grinding buffer (0.3 M sucrose, 50 mM Tris (pH 8), 5 mM magnesium chloride, 5 mM sodium bisulfite, 0.5 % NP40) and stirred on ice for 10 minutes. This mixture is filtered through six layers of cheesecloth, the liquid is transferred to centrifuge bottles and subjected to centrifugation at 700 x g for 10 minutes. The pellets are resuspended in grinding buffer and recentrifuged. The pellets are again resuspended in grinding buffer and this suspension is layered over a 20 ml cold sucrose cushion containing 10 mM Tris (pH 8), 1.5 mM magnesium chloride, 140 mM sodium chloride, 24 % sucrose, 1 % NP40. These tubes are centrifuged at 17,000 x g for 10 minutes. The pellets at this stage contained mostly nuclei and starch granules. High molecular weight DNA is isolated from the nuclei essentially according to Maniatis T et al - (1982). Etiolated plants are used as cleaner DNA is obtained with this extraction protocol.

2) Production of genomic libraries and isolation of genomic clones of green oat phytochrome.

The plant DNA is partially digested with MboI and ligated into the BamHI site of the Lambda Dash cloning vector. The phage are packaged using the Stratagene cloning system kit and a library with a primary titer of 2x10⁶ is created. The library is then amplified to a titer of 1x10¹⁰ phage/ml. To identify the phage containing green phytochrome, the plaques are lifted to Gene Screen plus (DuPont) and processed according to the manufacturers recommendations. The filters are prehybridized for four hours, and hybridized overnight at 42° C, in the manufacturer's hybridization solution containing 0.9M NaCl. The filters are then sequentially washed at a NaCl concentration of 0.9 M and monitored by autoradiography increasing the wash temperature by 5° C at each step until a constant hybridization pattern is obtained. Plaques are isolated that showed positive hybridization, replated and screened again with the probe mixture. This procedure is repeated until pure clones are obtained. Following purifi. cation of the phage clones DNA is isolated using the Lambda. sorb procedure of the Promega Kit following the manufacturers instructions. The Bluescript subclones are excised from the Lambda Zap library following the manufactureres protocol or subclones are created as described above.

The Lambda clones are then mapped by standard mapping procedures [Maniatis T *et al* (1982)] in order to identify unique clones. The fragments of the Lambda clones containing the sequences homologous to the oligonucleotide probe are then subcloned into Bluescript (Stratagene). The DNA sequence of the clones is obtained by a modification of the dideoxy sequencing protocol of Sanger *et al* (1977) using a double stranded template with the Sequenase Kit.

3) The Production of cDNA libraries and isolation of cDNA clones for green oat phytochrome.

Poly (A+) RNA is isolated by standard techniques and a cDNA library is constructed in the Lambda Zap cloning vector (Stratagene), essentially as described [Gubler U and Hoffmann BJ, (1983)]. 300,000 plaques are plated and duplicate plaque lifts are probed with the ³²P labeled oligonucleotide mixture 2 as described in section 1 above or by antibodies as described in section 4 below.

Once the green phytochrome cDNA clones are isolated, they are excised from Lambda Zap following the manufacturers protocol. They are then sequenced by double stranded dideoxy sequencing [Sanger *et al* (1977)].

4) Screening of cDNA libraries with antibodies to etiolated or green oat phytochrome.

Plaque lifts are immunologically screened as described by Young and Davis (1983). The plaque lifts are screened using polyclonal antibodies (pAb's) raised to the synthetic peptide described above and monoclonal antibodies (mAb's) GO-5, GO-7, and GO-8. Clones which are identified by cross-reaction with these polyclonal and monoclonal antibodies and which do not cross-react with mAb's O-3, O-13 and O-22 are characterized as described above.

C) Isolation of green phytochrome genes from other plant species.

1) DNA is isolated by the method of Murray & Thompson (1980) as modified by Taylor *et al* (1982) from Funk *Zea maize* line 2717; *Nicotiana tabaccum* cv xanthi, *Lycopersicum esculentum* cv UC82B, rice, wheat, and *Arabidopsis*.

2) Genomic libraries are prepared in Lambda Dash as described above and are screened with both the oligonucleotide mixture number one. Alternatively genomic libraries are screened with subclones of the oat genomic clone isolated above.

3) cDNA libraries are prepared from RNA isolated from the plants listed in C) 1). The phytochrome clones are isolated as described previously by hybridization with nucleic acid probes and antibodies to green oat phytochrome.

Bibliography

**Benjamin**, Inc., Menlo Park, CA (1977), pp. 356-357
**Berkelman** T and Lagarias JC, Anal. Biochem., 156: 194-201, (1986)

19

Bidlingmeyer *et al*, J. Chromato. Biomed. Appl., 336: 93-104 (1984)

Butler WL, Opt. Soc. Am.,52: 292-299, (1962)

Cordonnier M-M *et al*, Planta, 15: 369-376 (1983)

Cordonnier M-M *et al*, Biochemistry, 25: 7657-7666, (1986)

Gubler U. and Hoffmann BJ, Gene 25, 263, 1983

Haymes BD *et al*, In: Nucleic Acid Hybridization, A Practical Approach, IRL Press, Washington, DC (1985)

Johannson A *et al*, J. Immunol. Meth., 87: 7-11, (1986)

Köhler and Milstein, Nature, 256: 495-97 (1975)

Laemmli UK, Nature (London), 227: 680-685, (1970)

Lagarias JC *et al*, Photochem. Photobiol., 46: 5-13 (1987)

Lathe R *et al*, J. Molec. Biol. 183: 1-12 (1985)

Lehninger A, Biochemistry, Orth Publishers, New York, NY (1970)

Litts JC *et al*, J. Biol. Chem., 258: 11025-11031, (1983)

Maniatis T *et al*, Molecular Cloning A Laboratory Manual, Cold Spring Harbor Press, Cold Spring Harbor, NY (1982)

Murray & Thompson, Nuc. Acids. Res. 8: 4321 (1980)

Pratt LH, Plant Physiol., 51: 203-209 (1973)

Pratt LH Encycl. Plant Physiol.,New Ser., 16: 152-177 (1983)

Pratt LH, In: Techniques in Photomorphogenesis, Edited by Smith H and Holmes MG, pp. 175-200, Academic Press, London, New York, 1984

Pratt LH , Wampler JE and Rich ES Jr., Anal. Instrument. (N.Y.), 13: 269-287 (1985)

Pratt LH *et al*, In: Modern Methods of Plant Analysis, New Series, Vol. 4, Edited by Linskens HF and Jackson JF, pp. 50-74, Springer-Verlag, Berlin, 1986

Pratt LH and Cordonnier M-M, In: Phytochrome and Photoregulation in Plants, Edited by Furuya M, pp. 83-94, Academic Press, Tokyo, 1987

Rich ES Jr. and Wampler JE, Am. Lab. (Fairfield, Conn.), 14: 17-28, (1982)

Sanger *et al*, Proc. Natl. Acad. Sci., USA, 74: 5463 (1977)

Shimazaki Y and Pratt LH , Planta, 164: 333-344 (1985)

Taylor *et al*, BRL Focus, 4: 4-6 (1982)

Tokuhisa JG *et al*, Planta, 164: 321-332 (1985)

Towbin H *et al*, Proc. Natl. Acad. Sci. USA, 76: 4350-4354, (1979)

Wampler JE *et al*, Clin. Chem. (Winston-Salem, N.C.), 25: 1628-1634, (1979)

Young and Davis, Proc. Natl. Acad. Sci. USA, Vol. 8

## Claims

1. A hybridoma cell line that produces monoclonal antibodies that react selectively with a green-plant phytochrome and do not cross-react significantly with etiolated phytochrome from the same plant.

2. A hybridoma cell line of claim 1 wherein the plant is an oat plant.

3. A hybridoma cell line GO-5, GO-7 or GO-8 that produces monoclonal antibodies that react selectively with a green-plant phytochrome.

4. A monoclonal antibody that selectively binds to a green-plant phytochrome and does not cross-react significantly with etiolated phytochrome from the same plant.

5. A monoclonal antibody of claim 4 wherein the plant is an oat plant.

6. A monoclonal antibody produced by a hybridoma cell line identified as GO-5, GO-7 or GO-8.

7. A hybridoma cell line that produces monoclonal antibodies that react selectively with a green-plant phytochrome having the partial amino acid sequence:
V-A-E-I-T-G-L-P-T-M-E-A-I-G-M-P-L-V-D.

8. A hybridoma cell line of claim 7 wherein the plant is an oat plant.

9. The hybridoma cell line of claim 7 wherein said hybridoma cell line is identified as GO-5.

10. A monoclonal antibody that selectively binds to a green-plant phytochrome having the partial amino acid sequence:
V-A-E-I-T-G-L-P-T-M-E-A-I-G-M-P-L-V-D.

11. A monoclonal antibody of claim 10 wherein the plant is an oat plant.

12. A method for producing a hybridoma cell line that produces monoclonal antibodies that react selectively with a green-plant phytochrome comprising using as an immunogen a peptide comprising the amino acid sequence:

V-A-E-I-T-G-L-P-T-M-E-A-I-G-M-P-L-V-D.

13. A method for producing a hybridoma cell line that produces monoclonal antibodies that react selectively with a green-plant phytochrome comprising using as an immunogen a green-plant phytochrome or fragment thereof having the partial amino acid sequence:

V-A-E-I-T-G-L-P-T-M-E-A-I-G-M-P-L-V-D.

14. A method of claim 13 wherein the plant is an oat plant.

15. A hybridoma cell line that produces monoclonal antibodies that react selectively with a green-plant phytochrome, said hybridoma cell line produced by the method of anyone of claims 12 to 14.

16. A monoclonal antibody produced by a hybridoma cell line of claim 15.

17. A method for purifying a green-plant phytochrome suitable for use as an immunogen to produce hybridomas that react selectively with a green-plant phytochrome comprising purify ing a green-plant phytochrome by immunoaffinity chromatography utilizing monoclonal antibodies produced by at least one hybridoma selected from the group of hybridomas consisting of the hybridomas of anyone of claims 1 to 3.

18. A method for producing a hybridoma cell line that produces monoclonal antibodies that react selectively with a green-plant phytochrome comprising using as an immunogen the green-plant phytochrome purified by a method of claim 17.

19. A method of claim 17 or 18 wherein the plant is an oat plant.

20. A hybridoma cell line that produces monoclonal antibodies that react selectively with a green-plant phytochrome, said hybridoma cell line produced by the method of claim 18.

21. A hybridoma cell line of claim 20 wherein the plant is an oat plant.

22. A monoclonal antibody produced by a hybridoma cell line of claim 20 or 21.

23. An oligonucleotide probe comprising a base sequence selected from the group of base sequences derived from the following base sequence with random base insertions of permissible base substitutions as designated:

```
CCA ACA ATG GAG GCA ATT GGA ATG CC
..T ..T     ..A ..T ..C ..T
..C ..C         ..C ..A ..G
..G ..G         ..G     ..C
```

24. A mixture of oligonucleotide probes comprising base sequences derived from the following base sequence with random base insertions of permissible base substitutions as designated:

```
CCA ACA ATG GAG GCA ATT GGA ATG CC
..T ..T     ..A ..T ..C ..T
..C ..C         ..C ..A ..G
..G ..G         ..G     ..C
```

25. An oligonucleotide probe comprising a base sequence selected from the group of base sequences derived from the following base sequence with random base insertions permissible base substitutions as designated:

```
CCA ACA ATG GAG GCA ATT GGA ATG CC
..T ..T     ..A ..T ..C ..T
    ..C              ..G
                     ..C
```

26. A mixture of oligonucleotide probes comprising base sequences derived from the following base sequence with random base insertions of permissible base substitutions as designated:

CCA ACA ATG GAG GCA ATT GGA ATG CC
..T ..T     ..A ..T ..C ..T
   ..C           ..G
               ..C

27. Green-plant genomic DNA sequences that hybridize to an oligonucleotide probe of anyone of claims 23 to 26.

28. Green-plant genomic DNA sequence of claim 27, wherein the plant is an oat plant.

29. Green-plant phytochrome genes or fragments thereof that hybridize to an oligonucleotide probe of anyone of claims 23 to 26.

30. A green-plant phytochrome gene or fragment thereof of claim 29, wherein the plant is an oat plant.

31. Green-plant phytochrome promoter sequences associated with green-plant phytochrome genes that hybridize to an oligonucleotide probe of anyone of claims 23 to 26.

32. A green-plant phytochrome promoter gene of claim 31, wherein the plant is an oat plant.

33. Green-plant cDNA sequences that hybridize to an oligonucleotide probe of anyone of claims 23 to 26.

34. A green-plant cDNA sequence of claim 33, wherein the plant is an oat plant.

35. Green plant cDNA that encodes for green-plant phytochrome, said cDNA sequence capable of hybridizing with an oligonucleotide of anyone of claims 23 to 26.

36. A green-plant cDNA of claim 35, wherein the plant is an oat plant.

37. A method for cloning green-plant phytochrome genes or fragements thereof comprising screening genomic DNA from a green plant with an oligonucleotide probe of anyone of claims 23 to 26.

38. A method of claim 37 wherein the plant is an oat plant.

39. A method for cloning green-plant cDNA that encodes for green-plant phytochrome or a fragment thereof comprising screening the cDNA of a green plant with an oligonucleotide probe of anyone of claims 23 to 26.

40. A method of claim 39 wherein the plant is an oat plant.

41. A green-plant phytochrome gene or fragment thereof cloned by a method of claim 37 or 38.

42. A green-plant phytochrome gene or fragment thereof of claim 41 wherein he plant is an oat plant.

43. A green-plant cDNA that encodes for green-plant phytochrome or a fragment thereof, said cDNA being cloned by a method of claim 39 or 40.

44. A green-plant cDNA of claim 43 wherein the plant is an oat plant.

45. A method for cloning green-plant phytochrome gene promoter sequences comprising screening genomic DNA from a green plant with at least one probe selected from the group of probes consisting of oligonucleotide probes of anyone of claims 23 to 26, or DNA derived from anyone of claims 41 to 44.

46. A green-plant phytochrome gene promoter sequence cloned by a method of claim 45.

47. A method for purification of green-plant phytochrome that improves purity and eliminates immunodominant contaminants, which method comprises:
- extracting in a suitable extraction buffer plant material from green plants after homogenisation;
- fractionating the resulting crude phytochrome preparation by addition of poly (ethylenimine) and ammonium sulfate;
- further fractionating the resulting phytochrome preparation by hydroxyapatite (HA) chromatography;
- adding one ore more further ammonium sulfate fractionation steps; and
- electrophoresing the resulting phytochrome preparation.

48. A method according to claim 47 further comprising preparing multiple phytochrome preparations for fractionation by hydroxyapatite chromatography.

49. A method according to claim 47, wherein the crude phytochrome preparation is fractionated using ammonium sulfat not exceeding approximately 36% by volume of the final concentration of the total preparation; and the precipitate is dissolved in a small volume of a resuspension buffer.

50. A method according to claim 47 further comprising:
- adding a protease inhibitor to the phytochrome preparation;
- fractionating the phytochrome preparation using hydroxyapatite chromatography; and
- separating the peak fractions of the phytochrome preparation.

51. A method according to claim 50,wherein the protease inhibitor is iodoacetamide.

52. A method according to claim 47 further comprising:
- gel-electrophoresing the gree-plant phytochrome preparation to form a gel containing phytochrome;

- staining the gel with Coomassie blue; and
- excising phytochrome-containing bands.

53. A method according to claim 52 further comprising re-electrophoresing the phytochrome-containing bands of the gel after excision

54. A method according to claim 53 further comprising:
- electrotransferring the phytochrome preparation to nitrocellulose;
- visualizing the phytochrome preparation with $Zn^{2+}$-induced fluoreszence and Ponceau S stain; and
- excising the portion of nitrocellulose containing phytochrome.